# EUROPÄISCHE PATENTSCHRIFT

(11) **EP 1 820 496 B1**
(45) Veröffentlichungstag und Bekanntmachung des Hinweises auf die Patenterteilung: **12.10.2011**
(21) Anmeldenummer: 07001576.3
(22) Anmeldetag: 25.01.2007
(51) Int. Cl.: A61K 9/20, A61K 31/7036

(54) **Verfahren zur Herstellung einer medizinischen Vorrichtung zur Implantation**
Method of manufacturing a medical device for implantation
Procédé de fabrication d'un dispositif médical implantable

(30) Priorität: 15.02.2006 DE 102006007245
(43) Veröffentlichungstag der Anmeldung: 22.08.2007
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(56) Entgegenhaltungen:
- EP-A- 0 676 408
- DE-A1- 4 404 018
- VOGT S ET AL: "Resorbable antibiotic coatings for bone substitutes and implantable devices - Resorbierbare Antibiotische Beschichtungen fuer Knochenersatzwerkstoffe und Implantate" MATERIALWISSENSCHAFT UND WERKSTOFFTECHNIK, VCH VERLAGSGESELLSCHAFT, WEINHEIM, DE, Bd. 36, Nr. 12, 1. Dezember 2005 (2005-12-01), Seiten 814-819, XP002454072 ISSN: 0933-5137

## Beschreibung

Gegenstand der Erfindung ist ein Verfahren zur Herstellung einer biodegradierbaren medizinischen Vorrichtung zur Implantation, die als lokales Antibiotika-Depot im Rahmen der Behandlung der chronischen Osteomyelitis bestimmt ist.

Eine der schwierigsten Herausforderungen der Knochenchirurgie stellt auch gegenwärtig die Behandlung der Osteomyelitis dar. Osteomyelitis kann hämatogen, posttraumatisch oder postoperativ entstehen. Besonders schwierig ist die chronische Verlaufsform der Osteomyelitis zu behandeln, die im Extremfall zum Verlust von Gliedmaßen und bis zur Sepsis führen kann. Üblich ist bei der Behandlung der chronischen Osteomyelitis die chirurgische Sanierung durch radikales Debridment. Dabei wird der infizierte beziehungsweise nekrotische Knochen weiträumig abgetragen. Im Folgenden wird die Knochenkavität mit einem lokalen Antibiotika-Träger ausgefüllt oder durch eine Saug-Spül-Drainage behandelt. Durch die lokale Freisetzung hoher Antibiotika-Mengen aus einem Antibiotika-Träger lassen sich bei Verwendung eines hinreichend knochengängigen bakteriziden Antibiotikums, wie Gentamicinsulfat, auch die in den anliegenden Knochenarealen verbliebenen bakteriellen Keime wirksam bekämpfen.

Annähernd kugelförmige lokale Wirkstofffreisetzungssysteme, die aus Polymethylmethacrylat, Zirkoniumdioxid und einem konventionellen, in Wasser löslichem Antibiotikum, wie Gentamicinsulfat, aufgebaut sind, wurden erstmals 1975 von Klaus Klemm beschrieben (DE 23 20 373). Dieses Konzept erwies sich als erfolgreich, nachteilig war jedoch, dass nur ein geringer Teil des in den Kugeln enthaltenden Wirkstoffes freigesetzt wurde.

In Weiterentwicklung dieser Wirkstoffträger wurde 1978 von Heuser und Dingeldein vorgeschlagen, Glycin oder andere Aminosäuren zur Verbesserung der Antibiotikum-Freisetzung zuzusetzen (DE 26 51 441). Die inkorporierten Aminosäuren gehen nach Kontakt mit Blut beziehungsweise Wundsekret in Lösung und bilden Porensysteme, aus denen der Wirkstoff heraus diffundieren kann. Dadurch wurde eine verbesserte Wirkstofffreisetzung erreicht. Nach diesem Prinzip aufgebaute Wirkstoffträger sind gegenwärtig als perlschnurförmige Septopal^{®}-Ketten auf dem Markt. Dabei sind die Wirkstoffträger auf einen polyfilen Stahldraht aufgespritzt. Die retardierte Freisetzung beruht auf der Diffusion des Wirkstoffes aus der Polymermatrix. Der wesentliche Nachteil dieser Septopal^{®}-Ketten besteht darin, dass die Ketten im Allgemeinen nach ungefähr 10 Tagen wieder entfernt werden müssen. Dazu ist ein Zweiteingriff notwendig, der mit einer weiteren Belastung des Patienten verbunden ist und außerdem zusätzliche Kosten verursacht.

Es wurde im Folgenden angestrebt, ein vollständig biodegradierbares, perlschnurförmiges lokales Wirkstofffreisetzungssystem zur Verfügung zu stellen, um einen Zweiteingriff zur Entfernung des Wirkstofffreisetzungssystems zu vermeiden.

So wurde in DE 30 37 270 ein Wirkstoffträger beschrieben, der im Wesentlichen aus einem biodegradierbaren Faden besteht, auf dem Formkörper aus Fibrin angeordnet sind. In den Fibrin-Formkörpern ist ein Antibiotikum inkorporiert.

In US 5,756,127 wurde ein perlschnurförmiger Wirkstoffträger vorgeschlagen, bei dem Formkörper aus Calciumsulfat auf einem biodegradierbaren Faden befestigt sind. Das Calciumsulfat dient hierbei als Matrix für den Wirkstoff. Kritisch muss jedoch angemerkt werden, dass bei der Implantation größerer Mengen von Calciumsulfat mitunter eine Serom-Bildung beobachtet wurde.

DE 102 27 935 beschreibt lediglich mit Antibiotika-Fettsäuresalzen beschichtete, poröse Körper. In DE 101 14 244 A1 geht es um Gemische bestehend aus in Wasser leicht löslichen Antibiotika-Salzen und Salzen von amphiphilen Verbindungen (wie z.B. Alkylsulfonaten), die zusammen mit Hilfsstoffen zu Formkörpern geformt werden und direkt als antibiotisch wirksame Implantate dienen können. Wesentlich ist dabei, dass sich erst bei Kontakt mit Wasser beziehungsweise Körpertlüssigkeit innerhalb der Implantate in situ in Wasser gering lösliche Antibiotika-Salze durch reziproken Salzaustausch bilden.

DE 101 14 364 A1 beschreibt die Verwendung von Antibiotika-Fettsäuresalzen, -Organosulfaten oder -Organosulfonaten als Bindemittel zur Herstellung von Formkörpern, die organische oder anorganische Hilfsstoffe enthalten.

Es kann zusammenfassend festgestellt werden, dass die Grundidee der bei den Patenten DE 23 20 373, DE 26 51 441, DE 30 37 270 und US 5,756,127 vorgeschlagenen Wirkstofffreisetzungssysteme darin besteht, dass der Wirkstoff in einer Matrix eingebaut ist, aus welcher der Wirkstoff langsam infolge der Einwirkung von Blut beziehungsweise Wundsekret herausgelöst wird. Nachteilig an den aufgeführten Wirkstofffreisetzungssystemen ist, dass immer eine Matrix vorhanden ist, die entweder nur aufwendig gefertigt werden kann oder die auf Grund ihrer Zusammensetzung bei der Resorption und der damit zwangsläufig verbundenen Abbauprodukte unerwünschte Nebenwirkungen hervorrufen kann.

EP 0 676 408 A1 offenbart auf Fäden aufgereihte Antibiotika Formkörper, die Füllstoffe wie Hydroxylapatit/ZrO₂ enthalten können (Spalte 3). Die Formkörper werden laut Spalte 1 entweder durch Kristallisation zu einem Kuchen und anschließendes Zerteilen oder direkt durch Kristallisation in vorgegebenen geformten Kristallisationsgefäßen hergestellt.

Der Erfindung liegt die Aufgabe zu Grunde, eine implantierbare Vorrichtung zur Verfügung zu stellen, die als lokal anwendbares Antibiotika-Depot zur Behandlung der Osteomyelitis geeignet ist. Dabei sollen die Nachteile der bekannten Gentamicin enthaltenden perlschnurförmigen Wirkstofffreisetzungssysteme überwinden werden.

Die Aufgabe der Erfindung wird durch ein Verfahren zur Herstellung einer medizinischen Vorrichtung zur Implantation, bei der Formkörper, die aus mindestens einem Antibiotikasalz bestehen, das aus der Gruppe ausgewählt ist, die aus Gentamicinmyristat, Gentamicinpalmitat, Gentamicinstearat, Tobramycinmyristat, Tobramycinpalmitat, Tobramycinstearat, Amikacinmyristat, Amikacinpalmitat, Amikacinstearat, Vancomycinpalmitat, Vancomycinstearat, Ramoplaninpalmitat, Ramoplaninstearat, Levofloxacinpalmitat, Levofloxacinstearat, Ofloxacinpalmitat, Ofloxacinstearat, Moxifloxacinpalmitat, Moxifloxacinstearat, Clindamycinpalmitat und Clindamycinstearat besteht, auf einem biodegradierbaren Faden in einem Abstand von 1 mm bis 25 mm angeordnet sind, dadurch gekennzeichnet, dass dieses mindestens eine Antibiotikasalz auf den biodegradierbaren Faden gepresst und anschließend bei 50 - 70°C getempert wird , gelöst.

Unter den Begriffen Palmitat, Stearat und Myristat werden die Antibiotika-Salze der Palmitinsäure, der Stearinsäure und der Myristinsäure verstanden. Dabei ist das bevorzugte Stoffmengenverhältnis von protonierter Aminogruppe zu Fettsäure-Anion gleich 1. Es ist jedoch auch möglich, dass nur ein Teil der protonierten Aminogruppen Fettsäure-Anionen als Gegen-ionen be sitzen. So können zum Beispiel Gentamicinpentakispalmitat, Gentamicintetrakispalmitat oder auch Gentamicintripalmiat als in Wasser gering lösliche Antibiotika-Salze verwendet werden.

Bevorzugt ist die Herstellung einer derartigen medizinischen Vorrichtung, bei der entlang der Fadenachse 10, 20 oder 30 Formkörper angebracht sind. Für die Fäden eignet sich prinzipiell jedes resorbierbare Fadenmaterial. Es ist überraschend, dass mit den genannten in Wasser gering löslichen Antibiotika-Salzen ohne zusätzliche Verwendung von Matrixbildnem hinreichend stabile Formkörper hergestellt werden können. Obwohl es möglich ist, Hilfsstoffe vorzusehen, sind keine konventionellen anorganischen oder organischen Matrix-bildenden Hilfsstoffe erforderlich. Solche Hilfsstoffe wären z.B. Palmitinsäure, Myristinsäure, Stearinsäure, Glycerintripalmitat, Glycerintrimyristat oder Glycerintristearat, wobei der Hilfsstoffgehalt für gewöhnlich bis zu 90 Gewichtsprozent betragen kann.

Besonders bewährt hat sich das Verfahren zur Herstellung kugelförmiger Formkörper aus Gentamicinpalmitat (Aktivitätskoeffizent 251) mit einer Masse von jeweils 30 mg (entspricht 7,5 mg Gentamicinbase pro Formkörper), die zur Vermeidung einer Überdosierung im Abstand von jeweils 10 mm entlang der Fadenachse angeordnet sind. Der besondere Vorteil dieses Implantatmaterials besteht darin, dass sich die aus einem oder mehreren in Wasser gering löslichen Antibiotika-Salzen bestehenden Formkörper parallel zur Wirkstofffreisetzung auflösen und dass die einzelnen Formkörper zueinander durch den Faden auf Abstand gehalten werden. Dadurch ist eine mögliche Überdosierung deutlich erschwert. Die perlschnurförmige Anordnung erlaubt eine Ausfüllung größerer Knochenkavitäten mit einer relativ geringen Anzahl von Formkörpern. Ein wesentlicher Vorteil des erfindungsgemäß hergestellten Implantatmaterials besteht darin, dass keine Matrixbildner notwendig sind. Dadurch sind von vornherein Probleme mit eventuellen Abbauprodukten ausgeschlossen. Ein weiterer Vorteil besteht in der Verwendung von Antibiotika-Salzen, die geradzahlige Fettsäuren enthalten. Die geradzahligen Fettsäuren wie Palmitinsäure und Stearinsäure sind natürliche Bestandteile des humanen Organismus und werden durch β-Oxidation problemlos verstoffwechselt.

Der biodegradierbare Faden ist bevorzugt geflochten. Besonders gut haften die Formkörper auf geflochtenen Polyglycolid-Fäden.

Obwohl es möglich ist, den Formkörpern in Wasser leicht lösliche Antibiotika zuzusetzen, sind solche erfindungsgemäß bevorzugt nicht enthalten. Leicht lösliche Antibiotika sind z.B Gentamicinsulfat, Tobramycinsulfat, Amikacinsulfat, Levofloxacinhydrochlorid, Ofloxacinhydrochlorid, Moxifloxacinhydrochlorid und Clindamycinhydrochlorid. Die Inkorporation von in Wasser leicht löslichen Antibiotika bringt für gewöhnlich den Vorteil einer initial hohen Wirkstofffreisetzung in den ersten Stunden nach Einbringung des lmplantatmaterials in ein wässriges Milieu. Zum gleichen Zweck ist es möglich, weitere, in Wasser lösliche Antiinfektiva zuzusetzen.

Es wird gemäß der Erfindung ein perlschnurförmiges Implantatmaterial zur Verfügung gestellt, das ohne Verwendung einer Matrix auskommt.

Die Erfindung wird durch nachstehende Beispiele näher erläutert:

### Beispiel 1

Mit einer konventionellen Tablettiermaschine werden aus Gentamicinpalmitatpulver (Aktivitätskoeffizient 251) Oblongformkörper mit einer Masse von 35 mg (entspricht 8,8 mg Gentamicinbase) hergestellt. Es werden zwei Formkörper zur Bestimmung der Gentamicinfreisetzung entnommen und in 20 ml Phosphat-Puffer pH 7,4 bei 37 °C eingelagert. Täglich werden 15 ml Freisetzungsmedium zur Gentamicin-Gehaltsbestimmung entnommen und durch 15 ml frischen Phosphat-Puffer ersetzt. Die Gentamicin-Gehaltsbestimmung erfolgt mit einem TDX-Analyser der Firma Abbott. Die Ergebnisse sind in Tabelle 1 dargestellt.

### Beispiel 2

Mit einer speziell angefertigten Tablettiermaschine werden aus Gentamicinpalmitatpulver (Aktivitätskoeffizient 251) Oblongformkörper mit einer Masse von 30 mg (entspricht 7,5 mg Gentamicinbase) auf einen geflochtenen Polyglycolid-Faden in einem Abstand von jeweils 10-11 mm gepresst. Es werden zwei Formkörper zur Bestimmung der Gentamicinfreisetzung herausgeschnitten und in 20 ml Phosphat-Puffer pH 7,4 bei 37 °C eingelagert. Täglich werden 15 ml Freisetzungsmedium zur Gentamicin-Gehaltsbestimmung entnommen und durch 15 ml frischen Phosphat-Puffer ersetzt. Die Gentamicin-Gehaltsbestimmung erfolgt mit einem TDX-Analyser der Firma Abbott. Die Ergebnisse sind in Tabelle 1 dargestellt.

| Zeit [d] | Kumulierte Gentamicinbasefreisetzung [µg/Formkörper] | | | | | | | |
|---|---|---|---|---|---|---|---|---|
| | 1 | 2 | 3 | 4 | 7 | 8 | 9 | 10 |
| Formkörper Beispiel 1 | 1192 | 1759 | 2375 | 3641 | 3974 | 4294 | 4550 | 4773 |
| Formkörper Beispiel 2 | 1101 | 1696 | 2316 | 3581 | 3920 | 4255 | 4504 | 4760 |

## Patentansprüche

1. Verfahren zur Herstellung einer medizinischen Vorrichtung zur Implantation, bei der Formkörper, die aus mindestens einem Antibiotikasalz bestehen, das aus der Gruppe ausgewählt ist, die aus Gentamicinmyristat, Gentamicinpalmitat, Gentamicinstearat, Tobramycinmyristat, Tobramycinpalmitat, Tobramycinstearat, Amikacinmyristat, Amikacinpalmitat, Amikacinstearat, Vancomycinpalmitat, Vancomycinstearat, Ramoplaninpalmitat, Ramoplaninstearat, Levofloxacinpalmitat, Levofloxacinstearat, Ofloxacinpalmitat, Ofloxacinstearat, Moxifloxacinpalmitat, Moxifloxacinstearat, Clindamycinpalmitat und Clindamycinstearat besteht, auf einem biodegradierbaren Faden in einem Abstand von 1 mm bis 25 mm angeordnet sind, **dadurch gekennzeichnet, dass** dieses mindestens eine Antibiotikasalz auf den biodegradierbaren Faden gepresst und anschließend bei 50 - 70°C getempert wird.

2. Verfahren nach Anspruch 1, **dadurch gekennzeichnet, dass** der Faden geflochten ist.

3. Verfahren nach Anspruch 1, **dadurch gekennzeichnet, dass** die Formkörper irregulär geformt sind.

4. Verfahren nach Anspruch 1, **dadurch gekennzeichnet, dass** die Formkörper eine rotationssymmetrische Form aufweisen.

## Claims

1. A method for the manufacture of a medical device for implantation, in which form bodies consisting of at least one antibiotic salt selected from the group consisting of gentamycin myristate, gentamycin palmitate, gentamycin stearate, tobramycin myristate, tobramycin palmitate, tobramycin stearate, amikacin myristate, amikacin palmitate, amikacin stearate, vancomycin palmitate, vancomycin stearate, ramoplanin palmitate, ramoplanin stearate, levofloxacine palmitate, levofloxacine stearate, ofloxacine palmitate, ofloxacine stearate, moxifloxacine palmitate, moxifloxacine stearate, clindamycin palmitate, and clindamycin stearate, are arranged on a biodegradable thread at a distance of 1 mm to 25 mm, **characterised in that** said at least one antibiotic salt is pressed onto the biodegradable thread and subsequently temperature-treated at 50 - 70°C.

2. The method according to claim 1, **characterized in that** the thread is braided.

3. The method according to claim 1, **characterized in that** the form bodies are irregularly shaped.

4. The method according to claim 1, **characterized in that** the form bodies are rotationally symmetrical in shape.

## Revendications

1. Procédé de fabrication d'un dispositif médical pour implantation, dans lequel des corps moulés qui se composent d'au moins un sel d'antibiotique qui est sélectionné parmi le groupe qui se compose du myristate de gentamicine, palmitate de gentamicine, stéarate de gentamicine, myristate de tobramycine, palmitate de tobramycine, stéarate de tobramycine, myristate d'amicacine, palmitate d'amicacine, stéarate d'amicacine, palmitate de vancomycine, stéarate de vancomycine, palmitate de ramoplanine, stéarate de ramoplanine, palmitate de levofloxacine, stéarate de levofloxacine, palmitate d'ofloxacine, stéarate d'ofloxacine, palmitate de moxifloxacine, stéarate de moxifloxacine, palmitate de clindamycine et stéarate de clindamycine, sont disposés sur un fil biodégradable à une distance de 1 mm à 25 mm, **caractérisé en ce que** cet au moins un sel d'antibiotique est pressé sur le fil biodégradable, puis recuit à 50 à 70°C.

2. Procédé selon la revendication 1, **caractérisé en ce que** le fil est tressé.

3. Procédé selon la revendication 1, **caractérisé en ce que** les corps moulés sont moulés de manière irrégulière.

4. Procédé selon la revendication 1, **caractérisé en ce que** les corps moulés présentent une forme à symétrie de rotation.
